(19) Europäisches Patentamt
European Patent Office
Office européen des brevets

(11) Veröffentlichungsnummer: **0 361 157 B1**

(12) # EUROPÄISCHE PATENTSCHRIFT

(45) Veröffentlichungstag der Patentschrift: **02.03.94**

(51) Int. Cl.$^5$: **C07D 213/26**, C07D 213/30, C09K 19/34, C09K 19/42

(21) Anmeldenummer: **89116470.9**

(22) Anmeldetag: **06.09.89**

Die Akte enthält technische Angaben, die nach dem Eingang der Anmeldung eingereicht wurden und die nicht in dieser Patentschrift enthalten sind.

(54) **Phenylpyridine.**

(30) Priorität: **06.09.88 CH 3334/88**

(43) Veröffentlichungstag der Anmeldung:
**04.04.90 Patentblatt 90/14**

(45) Bekanntmachung des Hinweises auf die Patenterteilung:
**02.03.94 Patentblatt 94/09**

(84) Benannte Vertragsstaaten:
**CH DE FR GB IT LI NL**

(56) Entgegenhaltungen:
**EP-A- 0 240 320**
**WO-A-86/07085**
**US-A- 4 684 220**

(73) Patentinhaber: **F. HOFFMANN-LA ROCHE AG**
**Postfach 3255**
**CH-4002 Basel(CH)**

Patentinhaber: **NIOPIC MOSCOW RESEARCH AND PRODUCTION ASSOCIATION**
**B. Sadovaya 1/4**
**Moskau 103787(SU)**

(72) Erfinder: **Buchecker, Richard, Dr.**
**Felsenstrasse 10**
**CH-8008 Zürich(CH)**
Erfinder: **Pavluchenko, Assya I., Dr.**
**62 M. Zhukov Prospect**
**Flat 38**
**SU-123448 Moskau(SU)**
Erfinder: **Petrov, Vladimir F.**
**66 Khoroshevskoye Shosse**
**Flat 87**
**SU-123007 Moskau(SU)**
Erfinder: **Schadt, Martin, Dr.**
**Liestalerstrasse 77**
**CH-4411 Seltisberg(CH)**
Erfinder: **Smirnova, Natalia I.**
**13-1 Likhzchevskoye Shosse**
**Flat 89**
**SU-141700 Dolgoprudny Moskau Region(SU)**
Erfinder: **Titov, Victor T., Prof. Dr.**
**12 Festivalnaya Street**
**Flat 95**
**SU-125581 Moskau(SU)**

EP 0 361 157 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

(74) Vertreter: **Lederer, Franz, Dr. et al**
**Lederer, Keller & Riederer**
**Patentanwälte**
**Prinzregentenstrasse 16**
**D-80538 München (DE)**

## Beschreibung

Die vorliegende Erfindung betrifft neue 2-(2-Fluorphenyl)pyridine, deren Herstellung, flüssigkristalline Gemische, die diese Verbindungen enthalten, sowie die Verwendung für elektro-optische Zwecke.

Flüssige Kristalle werden vor allem als Dielektrika in Anzeigevorrichtungen verwendet, da die optischen Eigenschaften solcher Substanzen durch eine angelegte Spannung beeinflusst werden können. Elektrooptische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("super-birefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellungsspannungen und einen hohen Kontrast ergeben. Weiterhin sollten sie bei üblichen Betriebstemperturen, d.h. in einem möglichst breiten Bereich unterhalb und oberhalb Raumtemperatur eine geeignete Mesophase besitzen, beispielsweise für die oben genannten Zellen eine nematische oder cholesterische Mesophase. Daneben besteht in letzter Zeit auch ein vermehrtes Interesse an den besonders rasch schaltenden ferroelektrischen Flüssigkristallen mit chiral getilteter, smektischer Mesophase, insbesondere einer smektisch C Phase.

Da Flüssigkristalle in der Regel als Mischungen mehrerer Komponenten zur Anwendung gelangen, ist es wichtig, dass die Komponenten untereinander gut mischbar sind. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine möglichst geringe elektrische Leitfähigkeit aufweisen.

Gegenstand der Erfindung sind die Verbindungen der allgemeinen Formel

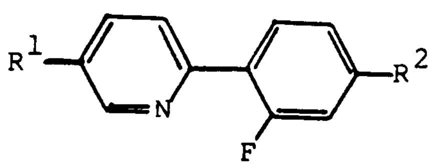

I

worin $R^1$ $(C_1\text{-}C_{18})$-Alkyl oder trans-$(C_1\text{-}C_{18}$ -Alkyl)cyclo-hexyl und $R^2$ $(C_1\text{-}C_{18})$-Alkyl, $(C_1\text{-}C_{18})$-Alkoxy oder trans-$(C_1\text{-}C_{18}$-Alkyl)cyclohexyl bedeuten.

Die erfindungsgemässen Verbindungen besitzen eine sehr hohe chemische und thermische Stabilität und eine hohe Stabilität gegenüber elektrischen Feldern und elektromagnetischer Strahlung. Sie sind vergleichsweise niederviskos, besitzen nur einen kleinen Absolutwert der dielektrischen Anisotropie, sind gut mischbar mit bekannten Flüssigkristallmaterialien und ermöglichen bei Anwendung in Flüssigkristallgemischen tiefe Betriebs- und Schwellen-Spannungen und eine hohe Multiplexierbarkeit.

Flüssigkristallkomponenten mit einem Stickstoff-Heterocyclus sind zum Beispiel in US Patent 4 684 220, EP-A-0 240 320 und WO-A-86/07 085 beschrieben.

Im Gegensatz zu bekannten, unpolaren Flüssigkristallkomponenten mit einem Stickstoff-Heterocyclus, werden bei den erfindungsgemässen Verbindungen hoch geordnete smektische Phasen meist vollständig unterdrückt und trotzdem vergleichsweise breite Mesophasebereiche erhalten. Die erfindungsgemässen Verbindungen besitzen daher, insbesondere wenn die Alkyl- und Alkoxyreste nicht zu lang sind (Summe der Kohlenstoffatome in beiden Alkylresten in $R^1$ und $R^2$ bzw. die Summe der Kohlenstoffatome im Alkylrest in $R^1$ und im Alkoxyrest in $R^2$ vorzugsweise höchstens etwa 12), meist eine reine nematische Phase oder im Falle der optisch aktiven Verbindungen eine cholesterische Phase. Sie eignen sich daher vor allem als unpolare Komponenten für nematische und cholesterische Flüssigkristallgemische. Die erfindungsgemässen Verbindungen sind ferner gut geeignet als Komponenten für ferroelektrische Gemische, wobei die Alkyl- und Alkoxyreste mit Vorteil etwas länger gewählt werden. Vorzugsweise beträgt die Summe der Kohlenstoffatome in den Alkylresten in $R^1$ und $R^2$ bzw. die Summe der Kohlenstoffatome im Alkylrest in $R^1$ und im Alkoxyrest in $R^2$ mindestens etwa 8. beispielsweise etwa 10-18.

Die optisch aktiven Verbindungen der Formel I induzieren eine vergleichsweise starke Verdrillung, d.h. kleine Ganghöhen (Pitch), in Flüssigkristallgemischen. Ferner eignen sie sich zur Induzierung einer spontanen Polarisation in ferroelektrischen Gemischen. Die induzierte Verdrillung und die induzierte spontane Polarisation sind in der Regel dann besonders gross, wenn $R^1$ Alkyl und $R^2$ Alkyl oder Alkoxy bedeuten und/oder das Chiralitätszentrum nahe am benachbarten Ring ist.

Die Verbindungen der Formel I, worin $R^1$ Alkyl und $R^2$ Alkyl oder Alkoxy bedeuten eignen sich insbesondere als niederviskose Dotierstoffe. Die Verbindungen der Formel I, worin $R^1$ und/oder $R^2$ trans-4-Alkylcyclohexyl bedeuten, besitzen hohe Klärpunkte und eignen sich u.a. zur Erhöhung der Klärpunkte von Gemischen.

Der obige Ausdruck "Alkyl" und der Alkylrest in "trans-4-Alkylcyclohexyl" und in "Alkoxy" umfassen jeweils geradkettige und verzeigte Reste mit vorzugsweise 1-18 Kohlenstoffatomen. Besonders bevorzugt sind Reste mit 1-12, insbesondere 1-7 Kohlenstoffatomen; für ferroelektrische Anwendungen können jedoch die Reste mit Vorteil auch länger sein und vorzugsweise etwa 6-14, besonders bevorzugt etwa 6-12 Kohlenstoffatome aufweisen. Im allgemeinen sind geradkettige Reste bevorzugt. Gewünschtenfalls können jedoch einer oder beide Reste verzweigt und gegebenenfalls chiral sein. Im Falle von verzweigten Gruppen steht der Ausdruck "Alkyl" bzw. der Alkylrest in "trans-4-Alkylcyclohexyl" und "Alkoxy" vorzugsweise für 1-Methylalkyl (= 2-Alkyl), 2-Methylalkyl oder Isoalkyl.

Gemäss Obigem bedeutet $R^1$ vorzugsweise $C_1$-$C_{18}$-Alkyl oder trans-4-($C_1$-$C_{18}$-Alkyl)cyclohexyl, besonders bevorzugt $C_1$-$C_{12}$-Alkyl oder trans-4-($C_1$-$C_{12}$-Alkyl)cyclohexyl, insbesondere $C_1$-$C_7$-Alkyl oder trans-4-($C_1$-$C_7$-Alkyl)cyclohexyl. Vorzugsweise ist der Alkylrest in $R^1$ jeweils geradkettig. $R^2$ bedeutet vorzugsweise $C_1$-$C_{18}$-Alkyl, $C_1$-$C_{18}$-Alkoxy oder trans-4-($C_1$-$C_{18}$-Alkyl)cyclohexyl, besonders bevorzugt $C_1$-$C_{12}$-Alkyl, $C_1$-$C_{12}$-Alkoxy oder trans-4-($C_1$-$C_{12}$-Alkyl)cyclohexyl, und insbesondere $C_1$-$C_7$-Alkyl, $C_1$-$C_7$-Alkoxy oder trans-4-($C_1$-$C_7$-Alkyl)cyclohexyl. Der Alkylrest in $R^2$ ist vorzugsweise geradkettig, wenn $R^2$ Alkyl oder trans-4-Alkylcyclohexyl bedeutet. Eine Alkoxygruppe $R^2$ kann geradkettig oder verzweigt sein. Bevorzugte verzweigte Alkoxygruppen sind 1-Methylalkoxy (= 2-Alkoxy) und 2-Methylalkoxy.

Beispiele bevorzugter Alkylgruppen sind Methyl, Aethyl, Propyl, Butyl, s-Butyl, Pentyl (= 1-Pentyl), 2-Pentyl (= 1-Methylbutyl), 2-Methylbutyl, Isopentyl, Hexyl (= 1-Hexyl), 2-Hexyl (= 1-Methylpentyl), 3-Methylpentyl, Heptyl, Octyl (= 1-Octyl), 2-Octyl (= 1-Methylheptyl), Nonyl, Decyl, Undecyl und Dodecyl. Beispiele bevorzugter Alkoxygruppen sind Methoxy, Aethoxy, Propyloxy, Butyloxy, s-Butyloxy, Pentyloxy (= 1-Pentyloxy), 2-Pentyloxy (= 1-Methylbutyloxy), 2-Methylbutyloxy, Hexyloxy (= 1-Hexyloxy), 2-Hexyloxy (= 1-Methylpentyloxy), 3-Methylpentyloxy, Heptyloxy, Octyloxy (= 1-Octyloxy), 2-Octyloxy (= 1-Methylheptyloxy), Nonyloxy, Decyloxy, Undecyloxy und Dodecyloxy. Beispiele bevorzugter trans-4-Alkylcyclohexylgruppen sind diejenigen, worin "Alkyl" für Methyl, Aethyl, Propyl, Butyl, s-Butyl, Pentyl, 2-Methylbutyl, Hexyl, 3-Methylpentyl, Heptyl oder Octyl steht. Die chiralen Gruppen können jeweils in R- oder S-Form vorliegen.

Eine bevorzugte Gruppe von erfindungsgemässen Verbindungen sind diejenigen, worin $R^2$ eine geradkettige oder eine verzweigte, gegebenenfalls chirale Alkoxygruppe bezeichnet. $R^1$ bedeutet in diesem Falle vorzugsweise n-Alkyl oder trans-4-n-Alkylcyclohexyl.

Die Verbindungen der Formel I können erfindungsgemäss dadurch hergestellt werden, dass man eine Verbindung der allgemeinen Formel

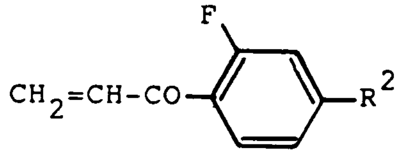

II

worin $R^2$ die obige Bedeutung hat,
mit einem Piperidin-Derivat der allgemeinen Formel

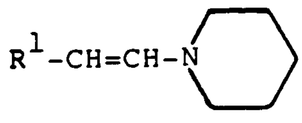

III

worin $R^1$ die obige Bedeutung hat,
und anschliessend mit Hydroxylamin in Gegenwart von Säure umsetzt.

Die Umsetzung kann in an sich bekannter Weise und unter üblichen Bedingungen erfolgen, beispielsweise nach der in den Synthesebeispielen angegebenen Methode.

Die Ausgangsmaterialien der Formel III sind bekannte oder Analoge bekannter Verbindungen. Die Herstellung der Ausgangsmaterialien der Formel II und III und deren Umsetzung zu Verbindungen der Formel I wird anhand der Schemata 1 und 2 veranschaulicht, worin $R^3$ Alkyl und $R^4$ Wasserstoff oder Alkyl bedeuten und $R^1$ und $R^2$ die obigen Bedeutungen haben:

Schema 1

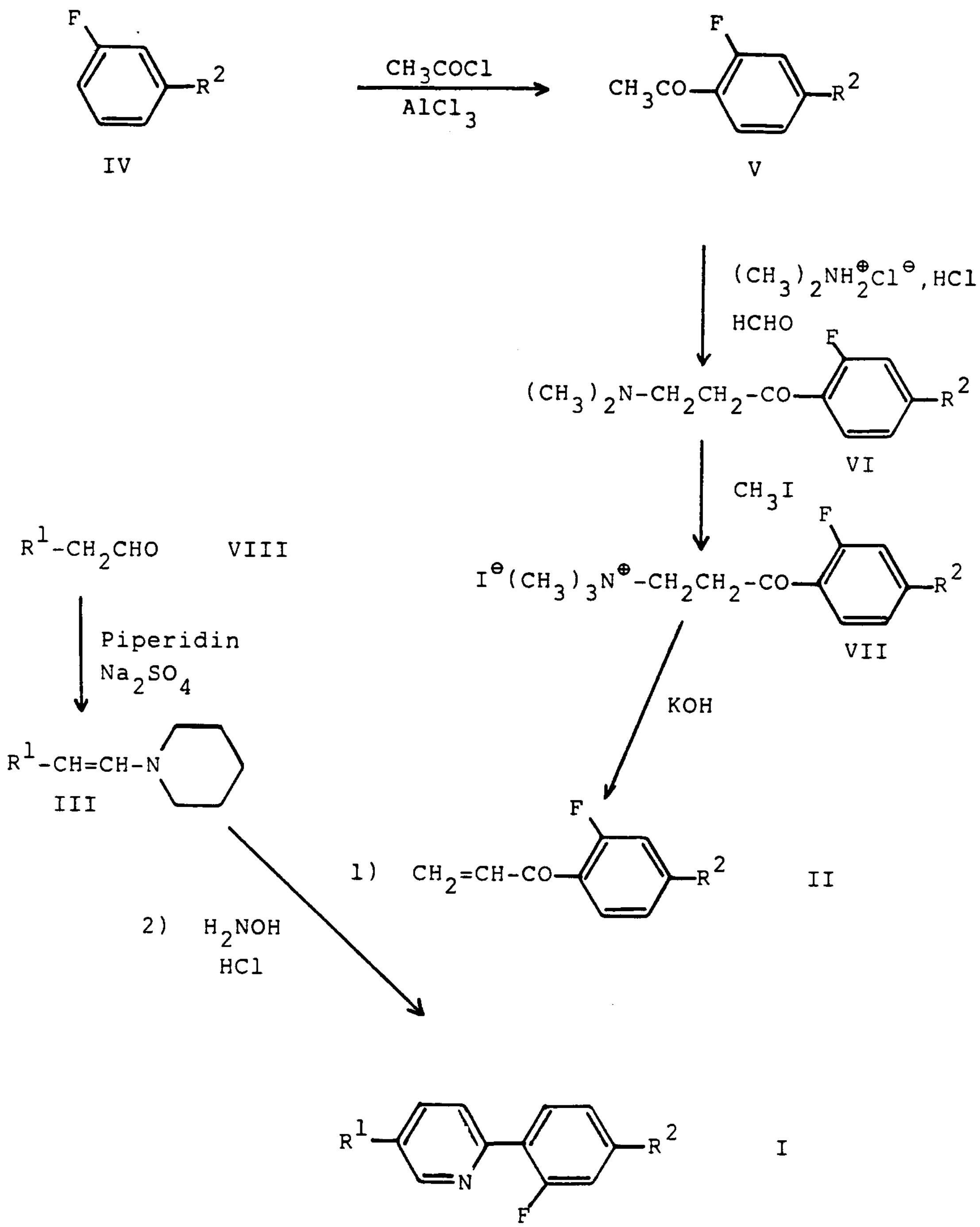
IV
$CH_3COCl$
$AlCl_3$
V
$(CH_3)_2NH_2^{\oplus}Cl^{\ominus}$, HCl
HCHO
VI
$CH_3I$
VII
VIII
Piperidin
$Na_2SO_4$
III
KOH
1)
II
2) $H_2NOH$
HCl
I

Schema 2

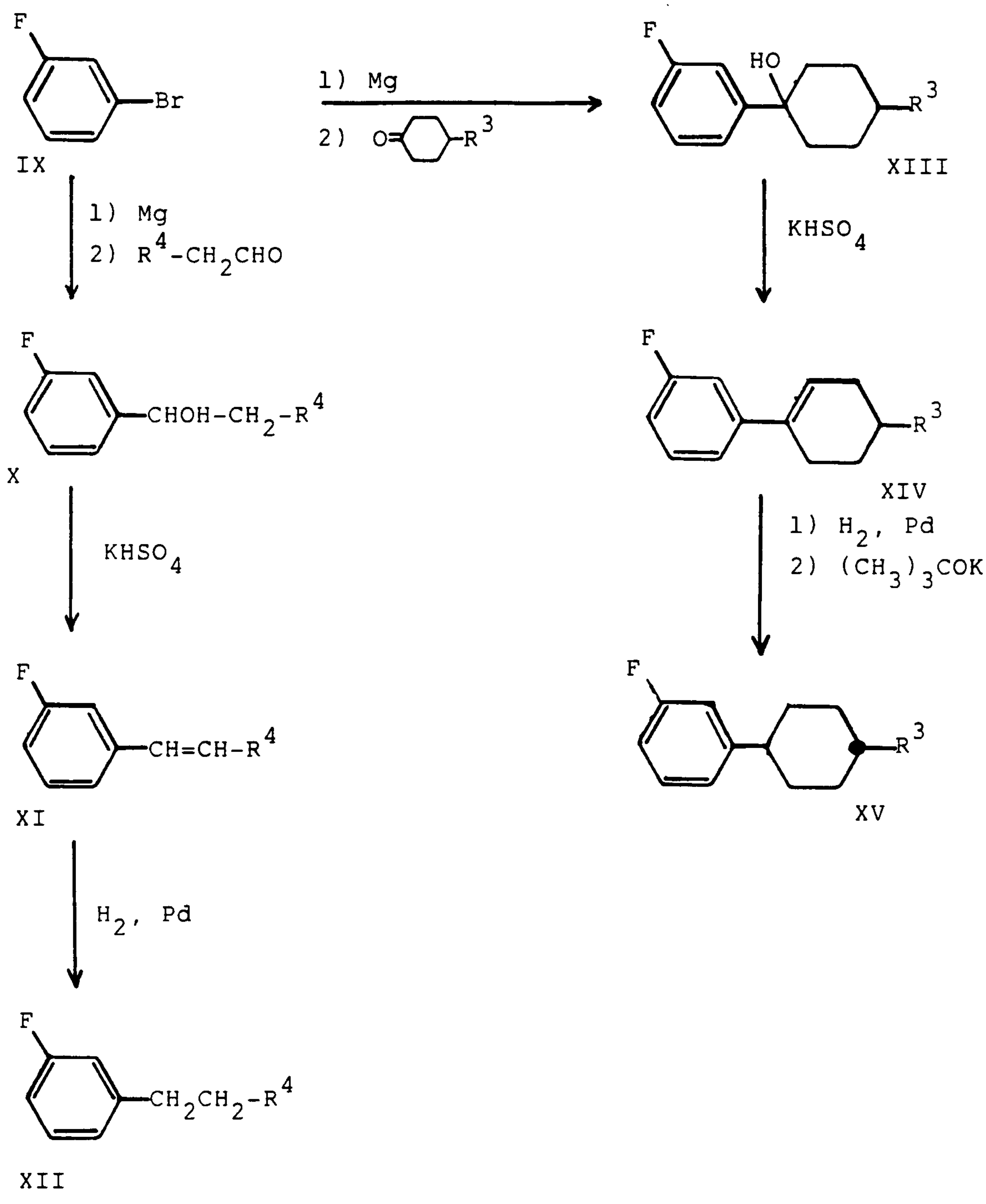


Die Ausgangsmaterialien der Formel VIII sind bekannte oder Analoge bekannter Verbindungen. Die Ausgangsmaterialien der Formel IV, worin $R^2$ Methyl oder Methoxy bedeutet, sind bekannt und im Handel erhältlich. Die Verbindungen der Formel IV, worin $R^2$ Alkoxy bedeutet, können aus dem bekannten m-Fluorphenol nach üblichen Methoden erhalten werden, beispielsweise durch Verätherung mit Alkylbromid in Gegenwart von Natriumcarbonat. Die Herstellung der Verbindungen der Formel IV, worin $R^2$ Alkyl oder trans-4-Alkylcyclohexyl bedeutet, kann gemäss der in Schema 2 illustrierten Methode erfolgen. Gewünschenfalls kann die Verbindung der Formel IX anstelle des Aldehyds der Formel $R^4$-$CH_2CHO$ auch mit einem Keton oder mit einem Aldehyd, der in α-Stellung eine Kettenverzweigung aufweist, umgesetzt werden.

Die Verbindungen der Formel I können in Form von Gemischen untereinander und/oder mit anderen Flüssigkristallkomponenten verwendet werden, wie z.B. mit Substanzen aus den Klassen der Schiffschen Basen, Azobenzole, Azoxybenzole, Phenylbenzoate, Cyclohexancarbonsäurephenylester, Cyclohexancarbonsäurecyclohexylester, Biphenyle, Phenylcyclohexane, Cyclohexylcyclohexane, Phenylpyrimidine, Cyclohexylpyrimidine, Phenyldioxane, 2-Cyclohexyl-1-phenyläthane, Terphenyle, Cyclohexylbiphenyle, Cyclohexylphenylpyrimidine und dergleichen. Derartige Substanzen sind dem Fachmann bekannt und viele davon sind zudem im Handel erhältlich.

Die erfindungsgemässen flüssigkristallinen Gemische enthalten mindestens zwei Komponenten, wovon mindestens eine Komponente eine Verbindung der Formel I ist. Eine zweite und gegebenenfalls weitere Komponenten können weitere Verbindungen der Formel I und/oder andere Flüssigkristallkomponenten sein.

Die Verbindungen der Formel I eignen sich insbesondere für nematische oder, sofern mindestens eine Komponente des Gemisches optisch aktiv ist, auch für cholesterische Gemische. Ein bevorzugter Anwendungsbereich betrifft die Verwendung als Dielektrikum in Flüssigkristallanzeigevorrichtungen mit verdrillt nematischer Flüssigkristallstruktur, wie TN-Zellen, STN-Zellen, SBE-Zellen und OMI-Zellen. Bevorzugte Gemische sind daher diejenigen, welche eine oder mehrere Verbindungen der Formel I und eine oder mehrere Verbindungen mit positiver dielektrischer Anisotropie enthalten. Ferner eignen sich die Verbindungen der Formel I als Komponenten chiral getilteter smektischer Phasen.

Der Anteil an Verbindungen der Formel I in den erfindungsgemässen Gemischen kann über einen relativ breiten Bereich variieren und beispielsweise etwa 1-60 Gew.-% betragen. Im allgemeinen ist ein Anteil von etwa 3-30 Gew.-%, insbesondere etwa 5-20 Gew.-% an Verbindungen der Formel I bevorzugt.

Vorzugsweise enthalten die erfindungsgemässen nematischen oder cholesterischen Gemische neben einer oder mehreren Verbindungen der Formel I eine oder mehrere Verbindungen aus der Gruppe der Verbindungen der allgemeinen Formeln

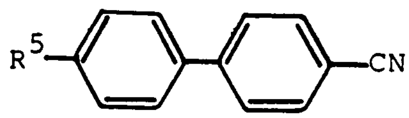

XVI

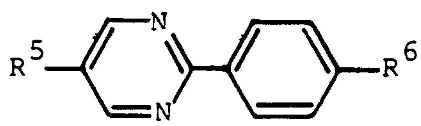

XVII

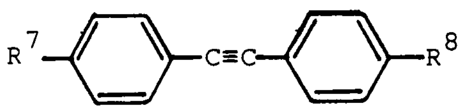

XVIII

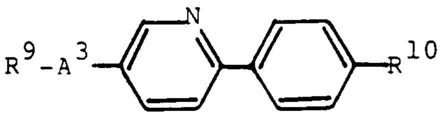

XIX

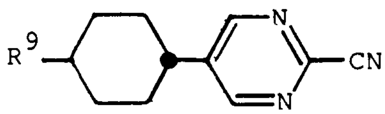

XX

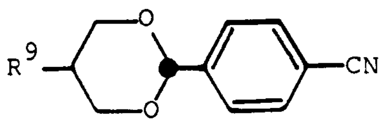

XXI

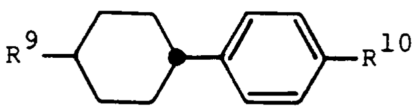

XXII

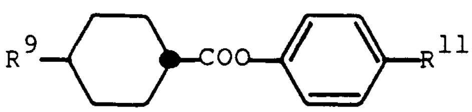

XXIII

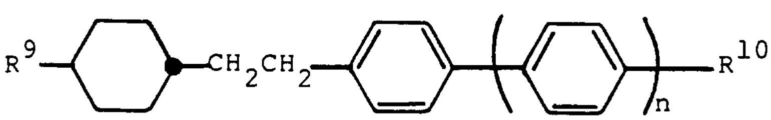

XXIV

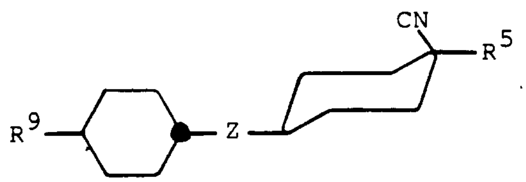

XXV

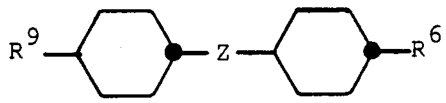

XXVI

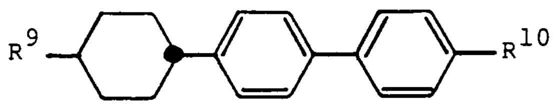

XXVII

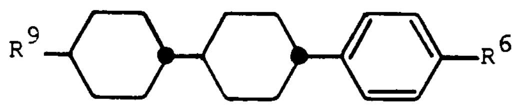

XXVIII

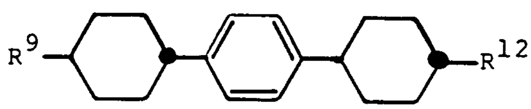

XXIX

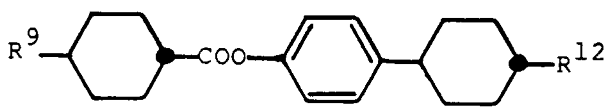

XXX

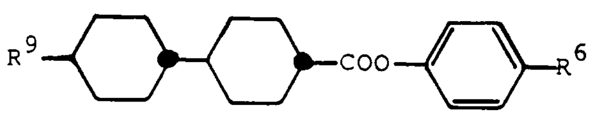

XXXI

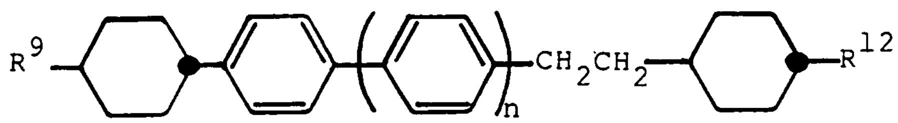

XXXII

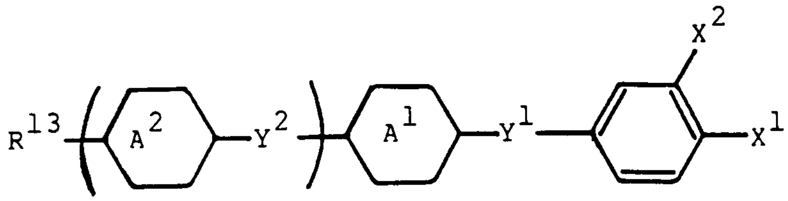

XXXIII

worin $R^5$ Alkyl, 3E-Alkenyl oder 4-Alkenyl bedeutet; $R^6$ Cyano, Alkyl, 1E-Alkenyl, 3E-Alkenyl, 4-Alkenyl, Alkoxy, 2E-Alkenyloxy oder 3-Alkenyloxy darstellt; $R^7$ und $R^8$ Alkyl oder Alkoxy bezeichnen; $R^9$ und $R^{12}$ unabhängig voneinander Alkyl, 1E-Alkenyl, 3E-Alkenyl oder 4-Alkenyl bedeuten; $A^3$ eine einfache Kovalenzbindung, 1,4-Phenylen oder trans-1,4-Cyclohexylen darstellt; $R^{10}$ Cyano, -NCS, Alkyl, 1E-Alkenyl, 3E-Alkenyl, 4-Alkenyl, Alkoxy, 2E-Alkenyloxy oder 3-Alkenyloxy bezeichnet; $R^{11}$ Alkoxy, 2E-Alkenyloxy oder 3-Alkenyloxy bedeutet; n für die Zahl 0 oder 1 steht; Z eine einfache Kovalenzbindung oder $-CH_2CH_2-$ darstellt; $X^1$ Fluor oder Chlor und $X^2$ Wasserstoff, Fluor oder Chlor bezeichnen; $R^{13}$ Alkyl, 3E-Alkenyl, 4-Alkenyl, Alkoxy, 2E-Alkenyloxy oder 3-Alkenyloxy bedeutet; eine der Gruppen $Y^1$ und $Y^2$ eine einfache Kovalenzbindung, -COO-, -OOC-, $-CH_2CH_2-$, $-CH_2O-$ oder $-OCH_2-$ und die andere der Gruppen $Y^1$ und $Y^2$ eine einfache Kovalenzbindung bedeutet; und die Ringe $A^1$ und $A^2$ unabhängig voneinander substituiertes oder unsubstituiertes trans-1,4-Cyclohexylen, in welchem gegebenenfalls 2 nicht benachbarte $CH_2$-Gruppen durch Sauerstoff ersetzt sind, oder substituiertes oder unsubstituiertes 1,4-Phenylen, in welchem gegebenenfalls 1 oder 2 $CH_2$-Gruppen durch Stickstoff ersetzt sind, darstellen.

Der Ausdruck "substituiertes oder unsubstituiertes trans-1,4-Cyclohexylen, in welchem gegebenenfalls 2 nicht benachbarte $CH_2$-Gruppen durch Sauerstoff ersetzt sind", umfasst insbesondere trans-1,4-Cyclohexylen und trans-m-Dioxan-2,5-diyl sowie Ringe, die mit in Flüssigkristallen üblichen Substituenten, wie Cyano, Methyl, Fluor oder Chlor, substituiert sind, beispielsweise 1-Cyano-trans-1,4-cyclohexylen oder 2-Methyl-trans-1,4-cyclohexylen.

Der Ausdruck "substituiertes oder unsubstituiertes 1,4-Phenylen, in welchem gegebenenfalls 1 oder 2 CH-Gruppen durch Stickstoff ersetzt sind", umfasst insbesondere 1,4-Phenylen, Pyridin-2,5-diyl, Pyrazin-2,5-diyl und Pyrimidin-2,5-diyl, sowie Ringe, die mit in Flüssigkristallen üblichen Substituenten, wie Cyano, Methyl, Fluor oder Chlor substituiert sind, beispielsweise 2-Cyano-1,4-phenylen, 2-Fluor-1,4-phenylen, 2-Chlor-1,4-phenylen oder 2-Methyl-1,4-phenylen.

Bevorzugte Mischungskomponenten mit positiver dielektrischer Anisotropie sind die Cyano- und Halogenverbindungen der Formeln XVI, XVII, XIX, XX, XXI, XXII, XXIV, XXVI, XXVII, XXVIII, XXXI und XXXIII. Vorzugsweise enthalten nematische und cholesterische Gemische mit positiver dielektrischer Anisotropie etwa 20-70 Gew.-%, insbesondere etwa 25-50 Gew.-% an einer oder mehreren dieser Verbindungen.

Die erfindungsgemässen Mischungen können ferner optisch aktive Verbindungen (z.B. optisch aktive 4'-Alkyl- oder 4'-Alkoxy-4-biphenylcarbonitrile) und/oder dichroitische Farbstoffe (z.B. Azo-, Azoxy- oder Anthrachinonfarbstoffe) enthalten. Der Anteil solcher Verbindungen wird durch die Löslichkeit, die gewünschte Ganghöhe, Farbe, Extinktion und dergleichen bestimmt. Im allgemeinen beträgt der Anteil optisch aktiver Verbindungen und dichroitischer Farbstoffe höchstens je etwa 10 Gew.-% im Gesamtgemisch.

Die erfindungsgemässen Gemische für smektische Anwendungen (insbesondere für getiltete smektische oder chiral getiltete smektisch Phasen) enthalten neben einer oder mehreren Verbindungen der Formel I vorzugsweise eine oder mehrere Verbindungen aus der Gruppe der Verbindungen der allgemeinen Formeln

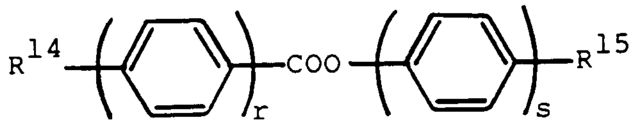

XXXIV

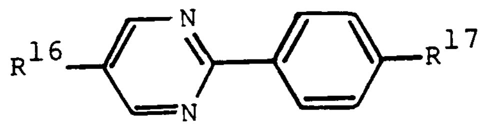

XXXV

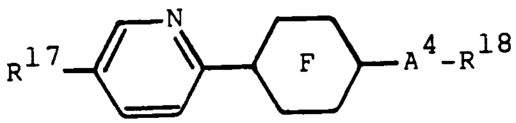

XXXVI

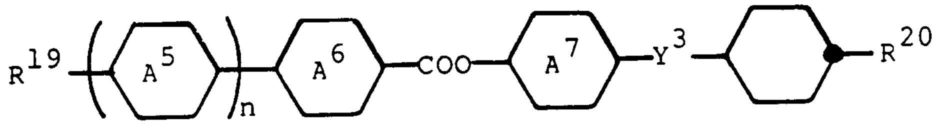

XXXVII

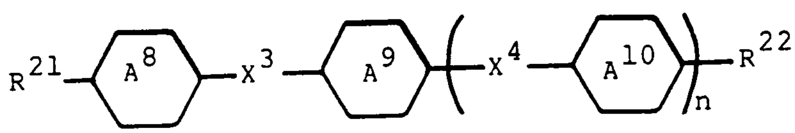

XXXVIII

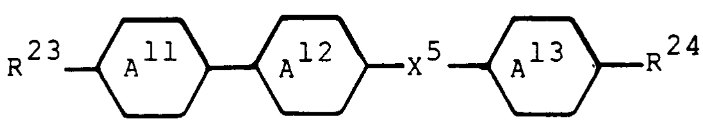

XXXIX

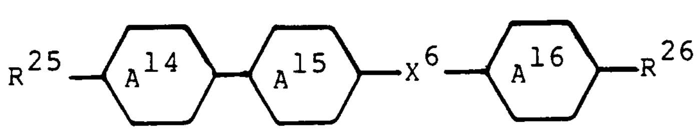

XL

worin $R^{14}$ und $R^{15}$ Alkyl, Alkoxy, Alkanoyl, Alkanoyloxy, Alkoxycarbonyl oder Alkoxycarbonyloxy mit bis zu 18 Kohlenstoffatomen bedeuten; r und s unabhängig voneinander 1 oder 2 bedeuten; $R^{16}$ und $R^{17}$ Alkyl oder Alkoxy mit 1-18 Kohlenstoffatomen darstellen; $A^4$ eine einfache Kovalenzbindung, trans-1,4-Cyclohexylen oder gegebenenfalls mit Halogen oder Methyl substituiertes 1,4-Phenylen bedeutet; Ring F trans-1,4-Cyclohexylen oder gegebenenfalls mit Halogen oder Methyl substituiertes 1,4-Phenylen darstellt; $R^{17}$ und $R^{18}$ je eine gegenbenenfalls halogensubstituierte Alkyl- oder Alkenylgruppe bezeichnen, in welcher gegebenenfalls eine oder zwei nicht benachbarte $CH_2$-Gruppen durch -O-, -COO-und/oder -OOC- ersetzt sind; n für

die Zahl 0 oder 1 steht; $Y^3$ eine einfache Kovalenzbindung, $-CH_2-CH_2-$, $-OCH_2-$, -COO- oder -OOC- bedeutet; die Ringe $A^5$, $A^6$ und $A^7$ gegebenenfalls mit Cyano, Halogen oder Niederalkyl substituiertes 1,4-Phenylen bezeichnen; $R^{19}$ und $R^{20}$ unabhängig voneinander gegebenenfalls halogensubstituiertes $C_1$-$C_{18}$-Alkyl oder gegebenenfalls halogensubstituiertes $C_2$-$C_{18}$-Alkenyl darstellen, in welchen gegebenenfalls eine oder zwei nicht benachbarte $CH_2$-Gruppen durch Sauerstoff ersetzt sind; $X^3$ eine einfache Kovalenzbindung, -COO- oder -OOC- und $X^4$ eine einfache Kovalenzbindung, -COO-, -OOC-, $-CH_2CH_2-$, $-OCH_2-$ oder $-CH_2O-$ darstellen; die Ringe $A^8$, $A^9$ und $A^{10}$ unabhängig voneinander unsubstituiertes oder mit Cyano, Halogen oder Niederalkyl substituiertes 1,4-Phenylen oder einer der Ringe auch Pyrimidin-2,5-diyl oder Pyrazin-2,5-diyl bedeutet und/oder, wenn n für die Zahl 1 steht, einer der Ringe auch trans-1,4-Cyclohexylen oder trans-m-Dioxan-2,5-diyl bedeutet; $R^{21}$ eine gegebenenfalls halogensubstituierte Alkenylgruppe mit bis zu 18 Kohlenstoffatomen bedeutet, worin gegebenenfalls 1 oder 2 nicht benachbarte $CH_2$-Gruppen durch -O-, -CO-, -COO- oder -OOC- ersetzt sind; $R^{22}$ eine gegebenenfalls halogensubstituierte Alkylgruppe mit bis zu 18 Kohlenstoffatomen bedeutet, worin gegebenenfalls 1 oder 2 nicht benachbarte $CH_2$-Gruppen durch -O-, -CO-, -COO- oder -OOC- ersetzt sind und/oder gegebenenfalls eine C-C-Einfachbindung durch eine C-C-Doppelbindung ersetzt ist; $X^5$ eine einfache Kovalenzbindung, -COO-, -OOC-, $-CH_2CH_2-$, $-OCH_2-$ oder $-CH_2O-$ bezeichnet; einer der Ringe $A^{11}$, $A^{12}$ und $A^{13}$ Pyrimidin-2,5-diyl darstellt, einer der Ringe $A^{11}$, $A^{12}$ und $A^{13}$ unsubstituiertes oder mit Cyano, Halogen oder Niederalkyl substituiertes 1,4-Phenylen darstellt und einer der Ringe $A^{11}$, $A^{12}$ und $A^{13}$ trans-1,4-Cyclohexylen oder unsubstituiertes oder mit Cyano, Halogen oder Niederalkyl substituiertes 1,4-Phenylen darstellt; und $R^{23}$ und $R^{24}$ unabhängig voneinander eine gegebenenfalls halogensubstituierte Alkylgruppe mit bis zu 18 Kohlenstoffatomen bedeuten, worin gegebenenfalls 1 oder 2 nicht benachbarte $CH_2$-Gruppen durch -O-, -CO-, -COO- und/oder -OOC- ersetzt sind; $X^5$ eine einfache Kovalenzbindung, -COO-, -OOC-, $-CH_2CH_2-$, $-OCH_2-$oder $-CH_2O-$ bezeichnet; einer der Ringe $A^{14}$, $A^{15}$ und $A^{16}$ trans-m-Dioxan-2,5-diyl darstellt, und die beiden anderen der Ringe $A^{14}$, $A^{15}$ und $A^{16}$ unabhängig voneinander unsubstituiertes oder mit Cyano, Halogen oder Niederalkyl substituiertes 1,4-Phenylen darstellen; $R^{25}$ und $R^{26}$ unabhängig voneinander eine gegebenenfalls halogensubstituierte Alkylgruppe mit bis zu 18 Kohlenstoffatomen bedeuten, worin gegebenenfalls 1 oder 2 nicht benachbarte $CH_2$-Gruppen durch -O-, -CO-, -COO- und/oder -OOC- ersetzt sind.

Die Herstellung der erfindungsgemässen Gemische und die Herstellung der elektro-optischen Vorrichtungen können in an sich bekannter Weise erfolgen.

Die Herstellung der Verbindungen der Formel I sowie flüssigkristalline Gemische enthaltend diese Verbindungen werden durch die folgenden Beispiele weiter veranschaulicht. C bedeutet eine kristalline, S eine smektische, $S_C$ eine smektisch C, N eine nematische, Ch eine cholesterische und I die isotrope Phase. $V_{10}$ bezeichnet die Spannung für 10% Transmission (Blickrichtung senkrecht zur Plattenoberfläche). $\Delta\epsilon = \epsilon_{\|} - \epsilon_{\perp}$ bezeichnet die dielektrische Anisotropie, wobei $\epsilon_{\|}$ die Dielektrizitätskonstante parallel zu den Moleküllängsachsen und $\epsilon_{\perp}$ die Dielektrizitätskonstante senkrecht dazu bedeutet. $\Delta n$ bezeichnet die optische Anisotropie.

Beispiel 1

0,078 Mol wasserfreies Natriumsulfat und 0,086 Mol Piperidin wurden gemischt und bei -5°C unter Stickstoff tropfenweise mit 0,039 Mol trans-4-Propylcyclohexylacetaldehyd versetzt. Das Reaktionsgemisch wurde 24 Stunden bei Raumtemperatur gerührt. Anschliessend wurde der Niederschlag abfiltriert und mit Hexan gewaschen. Destillation der vereinigten Filtrate unter Vakuum ergab 1-[2-(trans-4-Propylcyclohexyl)-vinyl]piperidin mit Sdp. 118-120°C/l Torr in 75% Ausbeute.

In analoger Weise können folgende Verbindungen hergestellt werden:

1-[2-(trans-4-Methylcyclohexyl)vinyl]piperidin;
1-[2-(trans-4-Aethylcyclohexyl)vinyl]piperidin, Sdp. 110-113°C/l Torr;
1-[2-(trans-4-Butylcyclohexyl)vinyl]piperidin;
1-[2-(trans-4-Pentylcyclohexyl)vinyl]piperidin, Sdp. 153-155°C/l Torr;
1-[2-(trans-4-Hexylcyclohexyl)vinyl]piperidin;
1-[2-(trans-4-Heptylcyclohexyl)vinyl]piperidin;
1-[2-(trans-4-Octylcyclohexyl)vinyl]piperidin;
1-(1-Propenyl)piperidin;
1-(1-Butenyl)piperidin;
1-(1-Pentenyl)piperidin;
1-(1-Hexenyl)piperidin;
1-(1-Heptenyl)piperidin;
1-(1-Octenyl)piperidin;

1-(1-Nonenyl)piperidin;
1-(1-Decyenyl)piperidin;
1-(1-Undecenyl)piperidin;
1-(1-Dodecenyl)piperidin.

Beispiel 2

a) Ein Gemisch von 0,13 Mol 1-Acetyl-2-fluor-4-methoxybenzol (herstellbar durch Verätherung von m-Fluorphenol mit Methylbromid in Gegenwart von Natriumcarbonat und anschliessende Acetylierung des 3-Fluor-1-methoxybenzols mit Acetylchlorid in Gegenwart von Aluminiumtrichlorid), 0,13 Mol Dimethylamin-hydrochlorid, Paraformaldehyd (0,22 Mol bezogen auf Formaldehyd-Gehalt), 25 ml wasserfreiem Isopropylalkohol und 0,02 ml konzentrierter Salzsäure wurde 8 Stunden gekocht. Anschliessend wurde das Reaktionsgemisch abgekühlt, mit 100 ml wasserfreiem Aceton verdünnt und filtriert. Der Rückstand von 1-[3-(Dimethylamino)propionyl]-2-fluor-4-methoxybenzol-hydrochlorid wurde in 200 ml Wasser gelöst. Die Lösung wurde mit 5-proz. (Gew./Vol.) Natronlauge auf pH 8 gestellt und mit Methylenchlorid extrahiert. Die organische Phase wurde mit wasserfreiem Natriumsulfat getrocknet und filtriert.

b) Die erhaltene Lösung von 1-[3-(Dimethylamino)propionyl]-2-fluor-4-methoxybenzol wurde unter Rühren bei 10-15 °C mit 0,15 Mol Methyljodid versetzt und 24 Stunden im Dunkeln aufbewahrt. Anschliessend wurde das feste 1-[3-(Dimethylamino)propionyl)-2-fluor -4-methoxybenzol-jodomethylat abfiltriert und mit Methylenchlorid gewaschen.

c) Das erhaltene 1-[3-(Dimethylamino)propionyl]-2-fluor-4-methoxybenzol-jodomethylat wurde in 220 ml Wasser und 360 ml Methylenchlorid suspendiert. Die Suspension wurde unter intensivem Rühren innert 15 Minuten mit einer Lösung von 6,4 g Kaliumhydroxid in 60 ml Wasser versetzt, wobei das feste Jodomethylat innert 15-20 Minuten verschwand. Die organische Phase wurde abgetrennt und die wässrige Phase wurde zweimal mit je 50 ml Methylenchlorid extrahiert. Die vereinigten organischen Extrakte wurden mit Wasser neutral gewaschen, über wasserfreiem Natriumsulfat getrocknet, filtriert und zur Stabilisierung mit wenig Hydrochinon versetzt. Nach Abtrennung des Lösungsmittels unter Vakuum wurde das erhaltene, instabile 1-Acrylyl-2-fluor-4-methoxybenzol ohne weitere Reinigung direkt in der nächsten Stufe (Beispiel 3) eingesetzt.

In analoge Weise können folgende Verbindungen hergestellt werden:
1-Acrylyl-2-fluor-4-äthoxybenzol;
1-Acrylyl-2-fluor-4-propyloxybenzol;
1-Acrylyl-2-fluor-4-butyloxybenzol;
1-Acrylyl-2-fluor-4-pentyloxybenzol;
1-Acrylyl-2-fluor-4-hexyloxybenzol;
1-Acrylyl-2-fluor-4-(2S-hexyloxy)benzol;
1-Acrylyl-2-fluor-4-heptyloxybenzol;
1-Acrylyl-2-fluor-4-octyloxybenzol;
1-Acrylyl-2-fluor-4-(2S-octyloxy)benzol;
1-Acrylyl-2-fluor-4-nonyloxybenzol;
1-Acrylyl-2-fluor-4-methylbenzol;
1-Acrylyl-2-fluor-4-äthylbenzol;
1-Acrylyl-2-fluor-4-propylbenzol;
1-Acrylyl-2-fluor-4-butylbenzol;
1-Acrylyl-2-fluor-4-pentylbenzol;
1-Acrylyl-2-fluor-4-hexylbenzol;
1-Acrylyl-2-fluor-4-heptylbenzol;
1-Acrylyl-2-fluor-4-(trans-4-methylcyclohexyl)benzol;
1-Acrylyl-2-fluor-4-(trans-4-äthylcyclohexyl)benzol;
1-Acrylyl-2-fluor-4-(trans-4-propylcyclohexyl)benzol;
1-Acrylyl-2-fluor-4-(trans-4-butylcyclohexyl)benzol;
1-Acrylyl-2-fluor-4-(trans-4-pentylcyclohexyl)benzol;
1-Acrylyl-2-fluor-4-(trans-4-hexylcyclohexyl)benzol;
1-Acrylyl-2-fluor-4-(trans-4-heptylcyclohexyl)benzol.

Beispiel 3

0,03 Mol 1-Acrylyl-2-fluor-4-methoxybenzol wurden bei 0 °C mit 0,03 Mol 1-[2-(trans-4-Propylcyclohexyl)vinyl]piperidin gemischt und 2 Stunden bei Raumtemperatur gehalten. Dann wurde das Gemisch mit 90 ml Aethanol, 10 ml Wasser, 0,14 Mol Hydroxylamin-hydrochlorid und 1 ml konzentrierter Salzsäure versetzt und 8 Stunden zum Rückfluss erhitzt. Anschliessend wurden durch Destillation ca. 2/3 des Lösungsmittels entfernt. Das eingeengte Gemisch wurde mit 150 ml Wasser verdünnt und mit wässriger Natriumcarbonat-Lösung auf pH 8 gestellt. Die organische Schicht wurde mit Benzol extrahiert. Der Extrakt wurde mit Wasser neutral gewaschen, über Natriumsulfat getrocknet, filtriert und eingeengt. Chromatographische Reinigung des erhaltenen Rückstandes an Kieselgel mit Benzol/Hexan (Vol.1:1) ergab 2-(4-Methoxy-2-fluorphenyl)-5-(trans -4-propylcyclohexyl)pyridin in 20% Ausbeute; Smp. (C-N) 56,3 °C Klp. (N-I) 180,6 °C.

In analoger Weise können folgende Verbindungen hergestellt werden:

2-(4-Methoxy-2-fluorphenyl)-5-methylpyridin;
2-(4-Methoxy-2-fluorphenyl)-5-äthylpyridin;
2-(4-Methoxy-2-fluorphenyl)-5-propylpyridin;
2-(4-Methoxy-2-fluorphenyl)-5-butylpyridin;
2-(4-Methoxy-2-fluorphenyl)-5-pentylpyridin, Smp. 23,3 °C;
2-(4-Methoxy-2-fluorphenyl)-5-hexylpyridin;
2-(4-Methoxy-2-fluorphenyl)-5-heptylpyridin;
2-(4-Aethoxy-2-fluorphenyl)-5-methylpyridin;
2-(4-Aethoxy-2-fluorphenyl)-5-äthylpyridin;
2-(4-Aethoxy-2-fluorphenyl)-5-propylpyridin;
2-(4-Aethoxy-2-fluorphenyl)-5-butylpyridin;
2-(4-Aethoxy-2-fluorphenyl)-5-pentylpyridin;
2-(4-Aethoxy-2-fluorphenyl)-5-hexylpyridin;
2-(4-Aethoxy-2-fluorphenyl)-5-heptylpyridin;
2-(4-Propyloxy-2-fluorphenyl)-5-methylpyridin;
2-(4-Propyloxy-2-fluorphenyl)-5-äthylpyridin;
2-(4-Propyloxy-2-fluorphenyl)-5-propylpyridin;
2-(4-Propyloxy-2-fluorphenyl)-5-butylpyridin;
2-(4-Propyloxy-2-fluorphenyl)-5-pentylpyridin;
2-(4-Propyloxy-2-fluorphenyl)-5-hexylpyridin;
2-(4-Butyloxy-2-fluorphenyl)-5-methylpyridin;
2-(4-Butyloxy-2-fluorphenyl)-5-äthylpyridin;
2-(4-Butyloxy-2-fluorphenyl)-5-propylpyridin;
2-(4-Butyloxy-2-fluorphenyl)-5-butylpyridin;
2-(4-Butyloxy-2-fluorphenyl)-5-pentylpyridin;
2-(4-Butyloxy-2-fluorphenyl)-5-hexylpyridin;
2-(4-Pentyloxy-2-fluorphenyl)-5-methylpyridin;
2-(4-Pentyloxy-2-fluorphenyl)-5-äthylpyridin;
2-(4-Pentyloxy-2-fluorphenyl)-5-propylpyridin;
2-(4-Pentyloxy-2-fluorphenyl)-5-butylpyridin;
2-(4-Pentyloxy-2-fluorphenyl)-5-pentylpyridin;
2-(4-Pentyloxy-2-fluorphenyl)-5-hexylpyridin;
2-(4-Hexyloxy-2-fluorphenyl)-5-methylpyridin;
2-(4-Hexyloxy-2-fluorphenyl)-5-äthylpyridin;
2-(4-Hexyloxy-2-fluorphenyl)-5-propylpyridin;
2-(4-Hexyloxy-2-fluorphenyl)-5-butylpyridin;
2-(4-Hexyloxy-2-fluorphenyl)-5-pentylpyridin;
2-(4-Hexyloxy-2-fluorphenyl)-5-hexylpyridin, Smp. (C-N) 16 °C, Klp. (N-I) 21,5 °C;
2-(4-Hexyloxy-2-fluorphenyl)-5-heptylpyridin;
2-(4-Hexyloxy-2-fluorphenyl)-5-octylpyridin;
2-(4-Hexyloxy-2-fluorphenyl)-5-nonylpyridin;
2-(4-Hexyloxy-2-fluorphenyl)-5-decylpyridin;
2-[4-(2S-Hexyloxy)-2-fluorphenyl]-5-methylpyridin;
2-[4-(2S-Hexyloxy)-2-fluorphenyl]-5-äthylpyridin;
2-[4-(2S-Hexyloxy)-2-fluorphenyl]-5-propylpyridin;
2-[4-(2S-Hexyloxy)-2-fluorphenyl]-5-butylpyridin;

2-[4-(2S-Hexyloxy)-2-fluorphenyl]-5-pentylpyridin;
2-[4-(2S-Hexyloxy)-2-fluorphenyl]-5-hexylpyridin;
2-[4-(2S-Hexyloxy)-2-fluorphenyl]-5-heptylpyridin;
2-[4-(2S-Hexyloxy)-2-fluorphenyl]-5-octylpyridin;
2-[4-(2S-Hexyloxy)-2-fluorphenyl]-5-nonylpyridin;
2-[4-(2S-Hexyloxy)-2-fluorphenyl]-5-decylpyridin;
2-(4-Heptyloxy-2-fluorphenyl)-5-propylpyridin;
2-(4-Heptyloxy-2-fluorphenyl)-5-butylpyridin;
2-(4-Heptyloxy-2-fluorphenyl)-5-pentylpyridin;
2-(4-Heptyloxy-2-fluorphenyl)-5-hexylpyridin;
2-(4-Heptyloxy-2-fluorphenyl)-5-heptylpyridin;
2-(4-Heptyloxy-2-fluorphenyl)-5-octylpyridin;
2-(4-Heptyloxy-2-fluorphenyl)-5-nonylpyridin;
2-(4-Heptyloxy-2-fluorphenyl)-5-decylpyridin;
2-(4-Octyloxy-2-fluorphenyl)-5-propylpyridin;
2-(4-Octyloxy-2-fluorphenyl)-5-butylpyridin;
2-(4-Octyloxy-2-fluorphenyl)-5-pentylpyridin;
2-(4-Octyloxy-2-fluorphenyl)-5-hexylpyridin;
2-(4-Octyloxy-2-fluorphenyl)-5-heptylpyridin;
2-(4-Octyloxy-2-fluorphenyl)-5-octylpyridin;
2-(4-Octyloxy-2-fluorphenyl)-5-nonylpyridin;
2-(4-Octyloxy-2-fluorphenyl)-5-decylpyridin;
2-[4-(2S-Octyloxy)-2-fluorphenyl]-5-pentylpyridin;
2-[4-(2S-Octyloxy)-2-fluorphenyl]-5-hexylpyridin;
2-[4-(2S-Octyloxy)-2-fluorphenyl]-5-heptylpyridin;
2-[4-(2S-Octyloxy)-2-fluorphenyl]-5-octylpyridin;
2-[4-(2S-Octyloxy)-2-fluorphenyl]-5-nonylpyridin;
2-[4-(2S-Octyloxy)-2-fluorphenyl]-5-decylpyridin;
2-(4-Nonyloxy-2-fluorphenyl)-5-octylpyridin;
2-(4-Nonyloxy-2-fluorphenyl)-5-nonylpyridin;
2-(4-Nonyloxy-2-fluorphenyl)-5-decylpyridin;
2-(4-Decyloxy-2-fluorphenyl)-5-heptylpyridin;
2-(4-Decyloxy-2-fluorphenyl)-5-octylpyridin;
2-(4-Decyloxy-2-fluorphenyl)-5-nonylpyridin;
2-(4-Methyl-2-fluorphenyl)-5-methylpyridin;
2-(4-Methyl-2-fluorphenyl)-5-äthylpyridin;
2-(4-Methyl-2-fluorphenyl)-5-propylpyridin;
2-(4-Methyl-2-fluorphenyl)-5-butylpyridin;
2-(4-Methyl-2-fluorphenyl)-5-pentylpyridin;
2-(4-Methyl-2-fluorphenyl)-5-hexylpyridin;
2-(4-Methyl-2-fluorphenyl)-5-heptylpyridin;
2-(4-Aethyl-2-fluorphenyl)-5-methylpyridin;
2-(4-Aethyl-2-fluorphenyl)-5-äthylpyridin;
2-(4-Aethyl-2-fluorphenyl)-5-propylpyridin;
2-(4-Aethyl-2-fluorphenyl)-5-butylpyridin;
2-(4-Aethyl-2-fluorphenyl)-5-pentylpyridin;
2-(4-Propyl-2-fluorphenyl)-5-methylpyridin;
2-(4-Propyl-2-fluorphenyl)-5-äthylpyridin;
2-(4-Propyl-2-fluorphenyl)-5-propylpyridin;
2-(4-Propyl-2-fluorphenyl)-5-butylpyridin;
2-(4-Propyl-2-fluorphenyl)-5-pentylpyridin;
2-(4-Butyl-2-fluorphenyl)-5-methylpyridin;
2-(4-Butyl-2-fluorphenyl)-5-äthylpyridin;
2-(4-Butyl-2-fluorphenyl)-5-propylpyridin;
2-(4-Pentyl-2-fluorphenyl)-5-methylpyridin;
2-(4-Pentyl-2-fluorphenyl)-5-äthylpyridin;
2-(4-Pentyl-2-fluorphenyl)-5-propylpyridin;
2-(4-Hexyl-2-fluorphenyl)-5-methylpyridin;

2-(4-Hexyl-2-fluorphenyl)-5-äthylpyridin;
2-(4-Hexyl-2-fluorphenyl)-5-propylpyridin;
2-(4-Heptyl-2-fluorphenyl)-5-methylpyridin;
2-(4-Heptyl-2-fluorphenyl)-5-äthylpyridin;
2-(4-Methoxy-2-fluorphenyl)-5-(trans-4-methylcyclohexyl)pyridin;
2-(4-Methoxy-2-fluorphenyl)-5-(trans-4-äthylcyclohexyl)pyridin, Smp. (C-N) 55,0 °C, Klp. (N-I) 155,0 °C;
2-(4-Methoxy-2-fluorphenyl)-5-(trans-4-butylcyclohexyl)pyridin;
2-(4-Methoxy-2-fluorphenyl)-5-(trans-4-pentylcyclohexyl)pyridin, Smp. (C-N) 44,6 °C, Klp. (N-I) 177,6 °C, $\Delta\epsilon$ (56 °C) = -0,4, $\epsilon_{\perp}$ (56 °C) = 3,68;
2-(4-Methoxy-2-fluorphenyl)-5-(trans-4-hexylcyclohexyl)pyridin;
2-(4-Methoxy-2-fluorphenyl)-5-(trans-4-heptylcyclohexyl)pyridin;
2-(4-Aethoxy-2-fluorphenyl)-5-(trans-4-methylcyclohexyl)pyridin;
2-(4-Aethoxy-2-fluorphenyl)-5-(trans-4-äthylcyclohexyl)pyridin;
2-(4-Aethoxy-2-fluorphenyl)-5-(trans-4-propylcyclohexyl)pyridin;
2-(4-Aethoxy-2-fluorphenyl)-5-(trans-4-butylcyclohexyl)pyridin;
2-(4-Aethoxy-2-fluorphenyl)-5-(trans-4-pentylcyclohexyl)pyridin;
2-(4-Propyloxy-2-fluorphenyl)-5-(trans-4-methylcyclohexyl)pyridin;
2-(4-Propyloxy-2-fluorphenyl)-5-(trans-4-äthylcyclohexyl)pyridin;
2-(4-Propyloxy-2-fluorphenyl)-5-(trans-4-propylcyclohexyl)pyridin;
2-(4-Butyloxy-2-fluorphenyl)-5-(trans-4-methylcyclohexyl)pyridin;
2-(4-Butyloxy-2-fluorphenyl)-5-(trans-4-äthylcyclohexyl)pyridin;
2-(4-Butyloxy-2-fluorphenyl)-5-(trans-4-propylcyclohexyl)pyridin;
2-(4-Pentyloxy-2-fluorphenyl)-5-(trans-4-methylcyclohexyl)pyridin;
2-(4-Pentyloxy-2-fluorphenyl)-5-(trans-4-äthylcyclohexyl)pyridin;
2-(4-Pentyloxy-2-fluorphenyl)-5-(trans-4-propylcyclohexyl)pyridin;
2-(4-Hexyloxy-2-fluorphenyl)-5-(trans-4-octylcyclohexyl)pyridin;
2-(4-Hexyloxy-2-fluorphenyl)-5-(trans-4-nonylcyclohexyl)pyridin;
2-(4-Hexyloxy-2-fluorphenyl)-5-(trans-4-decylcyclohexyl)pyridin;
2-[4-(2S-Hexyloxy)-2-fluorphenyl]-5-(trans-4-octylcyclohexyl)pyridin;
2-[4-(2S-Hexyloxy)-2-fluorphenyl]-5-(trans-4-nonylcyclohexyl)pyridin;
2-[4-(2S-Hexyloxy)-2-fluorphenyl]-5-(trans-4-decylcyclohexyl)pyridin;
2-(4-Heptyloxy-2-fluorphenyl)-5-(trans-4-heptylcyclohexyl)pyridin;
2-(4-Heptyloxy-2-fluorphenyl)-5-(trans-4-octylcyclohexyl)pyridin;
2-(4-Heptyloxy-2-fluorphenyl)-5-(trans-4-nonylcyclohexyl)pyridin;
2-(4-Heptyloxy-2-fluorphenyl)-5-(trans-4-decylcyclohexyl)pyridin;
2-(4-Octyloxy-2-fluorphenyl)-5-(trans-4-hexylcyclohexyl)pyridin;
2-(4-Octyloxy-2-fluorphenyl)-5-(trans-4-heptylcyclohexyl)pyridin;
2-(4-Octyloxy-2-fluorphenyl)-5-(trans-4-octylcyclohexyl)pyridin;
2-(4-Octyloxy-2-fluorphenyl)-5-(trans-4-nonylcyclohexyl)pyridin;
2-(4-Octyloxy-2-fluorphenyl)-5-(trans-4-decylcyclohexyl)pyridin;
2-[4-(2S-Octyloxy)-2-fluorphenyl]-5-(trans-4-hexylcyclohexyl)pyridin;
2-[4-(2S-Octyloxy)-2-fluorphenyl]-5-(trans-4-heptylcyclohexyl)pyridin;
2-[4-(2S-Octyloxy)-2-fluorphenyl]-5-(trans-4-octylcyclohexyl)pyridin;
2-[4-(2S-Octyloxy)-2-fluorphenyl]-5-(trans-4-nonylcyclohexyl)pyridin;
2-[4-(2S-Octyloxy)-2-fluorphenyl]-5-(trans-4-decylcyclohexyl)pyridin;
2-(4-Nonyloxy-2-fluorphenyl)-5-(trans-4-hexylcyclohexyl)pyridin;
2-(4-Nonyloxy-2-fluorphenyl)-5-(trans-4-heptylcyclohexyl)pyridin;
2-(4-Nonyloxy-2-fluorphenyl)-5-(trans-4-octylcyclohexyl)pyridin;
2-(4-Methyl-2-fluorphenyl)-5-(trans-4-methylcyclohexyl)pyridin;
2-(4-Methyl-2-fluorphenyl)-5-(trans-4-äthylcyclohexyl)pyridin;
2-(4-Methyl-2-fluorphenyl)-5-(trans-4-propylcyclohexyl)pyridin;
2-(4-Methyl-2-fluorphenyl)-5-(trans-4-butylcyclohexyl)pyridin;
2-(4-Methyl-2-fluorphenyl)-5-(trans-4-pentylcyclohexyl)pyridin;
2-(4-Aethyl-2-fluorphenyl)-5-(trans-4-methylcyclohexyl)pyridin;
2-(4-Aethyl-2-fluorphenyl)-5-(trans-4-äthylcyclohexyl)pyridin;
2-(4-Aethyl-2-fluorphenyl)-5-(trans-4-propylcyclohexyl)pyridin;
2-(4-Aethyl-2-fluorphenyl)-5-(trans-4-butylcyclohexyl)pyridin;
2-(4-Aethyl-2-fluorphenyl)-5-(trans-4-pentylcyclohexyl)pyridin;

2-(4-Propyl-2-fluorphenyl)-5-(trans-4-methylcyclohexyl)pyridin;
2-(4-Propyl-2-fluorphenyl)-5-(trans-4-äthylcyclohexyl)pyridin;
2-(4-Propyl-2-fluorphenyl)-5-(trans-4-propylcyclohexyl)pyridin;
2-(4-Propyl-2-fluorphenyl)-5-(trans-4-butylcyclohexyl)pyridin;
2-(4-Propyl-2-fluorphenyl)-5-(trans-4-pentylcyclohexyl)pyridin;
2-(4-Butyl-2-fluorphenyl)-5-(trans-4-methylcyclohexyl)pyridin;
2-(4-Butyl-2-fluorphenyl)-5-(trans-4-äthylcyclohexyl)pyridin;
2-(4-Butyl-2-fluorphenyl)-5-(trans-4-propylcyclohexyl)pyridin;
2-(4-Pentyl-2-fluorphenyl)-5-(trans-4-methylcyclohexyl)pyridin;
2-(4-Pentyl-2-fluorphenyl)-5-(trans-4-äthylcyclohexyl)pyridin;
2-(4-Pentyl-2-fluorphenyl)-5-(trans-4-propylcyclohexyl)pyridin;
2-[4-(trans-4-Methylcyclohexyl)-2-fluorphenyl]-5-methyl-pyridin;
2-[4-(trans-4-Methylcyclohexyl)-2-fluorphenyl]-5-äthyl-pyridin;
2-[4-(trans-4-Methylcyclohexyl)-2-fluorphenyl]-5-propyl-pyridin;
2-[4-(trans-4-Methylcyclohexyl)-2-fluorphenyl]-5-butyl-pyridin;
2-[4-(trans-4-Methylcyclohexyl)-2-fluorphenyl]-5-pentyl-pyridin;
2-[4-(trans-4-Aethylcyclohexyl)-2-fluorphenyl]-5-methyl-pyridin;
2-[4-(trans-4-Aethylcyclohexyl)-2-fluorphenyl]-5-äthyl-pyridin;
2-[4-(trans-4-Aethylcyclohexyl)-2-fluorphenyl]-5-propyl-pyridin;
2-[4-(trans-4-Aethylcyclohexyl)-2-fluorphenyl]-5-butylpyridin;
2-[4-(trans-4-Aethylcyclohexyl)-2-fluorphenyl]-5-pentyl-pyridin;
2-[4-(trans-4-Propylcyclohexyl)-2-fluorphenyl]-5-methyl-pyridin;
2-[4-(trans-4-Propylcyclohexyl)-2-fluorphenyl]-5-äthyl-pyridin;
2-[4-(trans-4-Propylcyclohexyl)-2-fluorphenyl]-5-propyl-pyridin;
2-[4-(trans-4-Propylcyclohexyl)-2-fluorphenyl]-5-butyl-pyridin;
2-[4-(trans-4-Propylcyclohexyl)-2-fluorphenyl]-5-pentyl-pyridin;
2-[4-(trans-4-Butylcyclohexyl)-2-fluorphenyl]-5-methyl-pyridin;
2-[4-(trans-4-Butylcyclohexyl)-2-fluorphenyl]-5-äthyl-pyridin;
2-[4-(trans-4-Butylcyclohexyl)-2-fluorphenyl]-5-propyl-pyridin;
2-[4-(trans-4-Pentylcyclohexyl)-2-fluorphenyl]-5-methyl-pyridin;
2-[4-(trans-4-Pentylcyclohexyl)-2-fluorphenyl]-5-äthyl-pyridin;
2-[4-(trans-4-Pentylcyclohexyl)-2-fluorphenyl]-5-propyl-pyridin;
2-[4-(trans-4-Methylcyclohexyl)-2-fluorphenyl]-5-(trans-4-methylcyclohexyl)pyridin;
2-[4-(trans-4-Methylcyclohexyl)-2-fluorphenyl]-5-(trans-4-äthylcyclohexyl)pyridin;
2-[4-(trans-4-Methylcyclohexyl)-2-fluorphenyl]-5-(trans-4-propylcyclohexyl)pyridin;
2-[4-(trans-4-Methylcyclohexyl)-2-fluorphenyl]-5-(trans-4-butylcyclohexyl)pyridin;
2-[4-(trans-4-Methylcyclohexyl)-2-fluorphenyl]-5-(trans-4-pentylcyclohexyl)pyridin;
2-[4-(trans-4-Aethylcyclohexyl)-2-fluorphenyl]-5-(trans-4-methylcyclohexyl)pyridin;
2-[4-(trans-4-Aethylcyclohexyl)-2-fluorphenyl]-5-(trans-4-äthylcyclohexyl)pyridin;
2-[4-(trans-4-Aethylcyclohexyl)-2-fluorphenyl]-5-(trans-4-propylcyclohexyl)pyridin;
2-[4-(trans-4-Aethylcyclohexyl)-2-fluorphenyl]-5-(trans-4-butylcyclohexyl)pyridin;
2-[4-(trans-4-Aethylcyclohexyl)-2-fluorphenyl]-5-(trans-4-pentylcyclohexyl)pyridin;
2-[4-(trans-4-Propylcyclohexyl)-2-fluorphenyl]-5-(trans-4-methylcyclohexyl)pyridin;
2-[4-(trans-4-Propylcyclohexyl)-2-fluorphenyl]-5-(trans-4-äthylcyclohexyl)pyridin;
2-[4-(trans-4-Propylcyclohexyl)-2-fluorphenyl]-5-(trans-4-propylcyclohexyl)pyridin;
2-[4-(trans-4-Propylcyclohexyl)-2-fluorphenyl]-5-(trans-4-butylcyclohexyl)pyridin;
2-[4-(trans-4-Propylcyclohexyl)-2-fluorphenyl]-5-(trans-4-pentylcyclohexyl)pyridin;
2-[4-(trans-4-Butylcyclohexyl)-2-fluorphenyl]-5-(trans-4-methylcyclohexyl)pyridin;
2-[4-(trans-4-Butylcyclohexyl)-2-fluorphenyl]-5-(trans-4-äthylcyclohexyl)pyridin;
2-[4-(trans-4-Butylcyclohexyl)-2-fluorphenyl]-5-(trans-4-propylcyclohexyl)pyridin;
2-[4-(trans-4-Pentylcyclohexyl)-2-fluorphenyl]-5-(trans-4-methylcyclohexyl)pyridin;
2-[4-(trans-4-Pentylcyclohexyl)-2-fluorphenyl]-5-(trans-4-äthylcyclohexyl)pyridin;
2-[4-(trans-4-Pentylcyclohexyl)-2-fluorphenyl]-5-(trans-4-propylcyclohexyl)pyridin.

Beispiel 4

Zur Illustration der erfindungsgemässen Gemische wurde die folgende Mischung hergestellt und deren elektro-optischen Daten in eine TN-Zelle bei Raumtemperatur gemessen:

Mischung A

| | | |
|---|---|---|
| 19 Gew.-% | | 5-Propyl-2-(4-cyanophenyl)pyridin, |
| 29 | " | 5-Pentyl-2-(4-cyanophenyl)pyridin, |
| 26 | " | trans-4-Butylcyclohexancarbonsäure-4-äthoxyphenylester, |
| 16 | " | trans-4-Hexylcyclohexancarbonsäure-4-äthoxyphenylester, |
| 10 | " | 2-(4-Methoxy-2-fluorphenyl)-5-(trans-4-pentylcyclohexyl)pyridin; |

Phasenübergang (S-N) <-20 °C, Klp. (N-I) 63,5 °C; $\Delta\epsilon$ = 11,5, $V_{10}$ = 1,52 V, $\Delta n$ = 0,162.

## Patentansprüche

1. Verbindungen der allgemeinen Formel

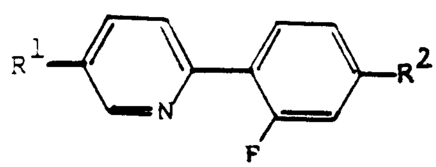

I

worin $R^1$ ($C_1$-$C_{18}$)-Alkyl oder trans-4-($C_1$-$C_{18}$-Alkyl)cyclohexyl und $R^2$ ($C_1$-$C_{18}$)-Alkyl, ($C_1$-$C_{18}$)-Alkoxy oder trans-4-($C_1$-$C_{18}$-Alkyl)cyclohexyl bedeuten.

2. Verbindungen der Formel I nach Anspruch 1, dadurch gekennzeichnet, dass $R^1$ ($C_1$-$C_{12}$)-Alkyl oder trans-4-($C_1$-$C_{12}$-Alkyl)cyclohexyl bedeutet.

3. Verbindungen der Formel I nach Anspruch 1 oder 2, dadurch gekennzeichnet, dass $R^2$ ($C_1$-$C_{12}$)-Alkyl, ($C_1$-$C_{12}$)-Alkoxy oder trans-4-($C_1$-$C_{12}$-Alkyl)-cyclohexyl bedeutet.

4. Verbindungen der Formel I nach einem der Ansprüche 1, 2 oder 3, dadurch gekennzeichnet, dass $R^1$ ($c_1$-$c_7$)-Alkyl oder trans-4-($C_1$-$C_7$-Alkyl)cyclohexyl und $R^2$ ($C_1$-$C_7$)-Alkoxy bedeuten.

5. Flüssigkristallines Gemisch mit mindestens 2 Komponenten, dadurch gekennzeichnet, dass mindestens eine Komponente eine Verbindung der in Anspruch 1 definierten Formel I ist.

6. Flüssigkristallines Gemisch nach Anspruch 5, dadurch gekennzeichnet, dass es 1-60 Gew.-% an Verbindungen der in Anspruch 1 definierten Formel I enthält.

7. Nematisches oder cholesterisches Gemisch nach Anspruch 5 oder 6, dadurch gekennzeichnet, dass es eine oder mehrere Verbindungen der Formel I und eine oder mehrere Verbindungen mit positiver dielektrischer Anisotropie enthält.

8. Nematisches oder cholesterisches Gemisch nach einem der Ansprüche 5 bis 7, dadurch gekennzeichnet, dass es eine oder mehrere Verbindungen der der in Anspruch 1 definierten Formel I und eine oder mehrere Verbindungen aus der Gruppe der Verbindungen der allgemeinen Formeln

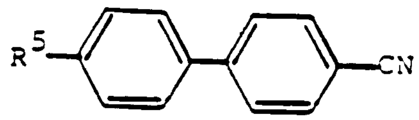

XVI

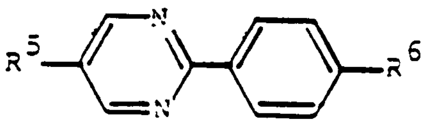

XVII

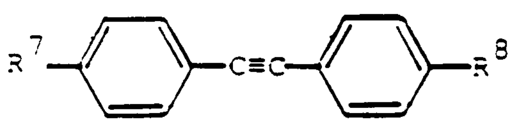

XVIII

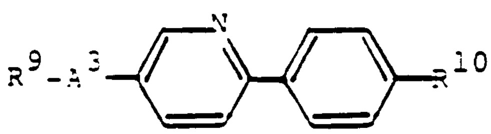

XIX

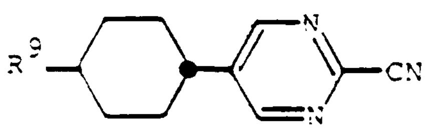

XX

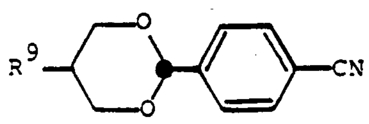

XXI

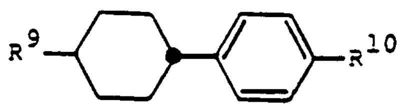

XXII

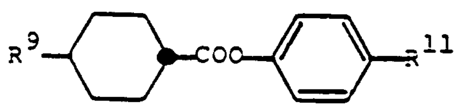

XXIII

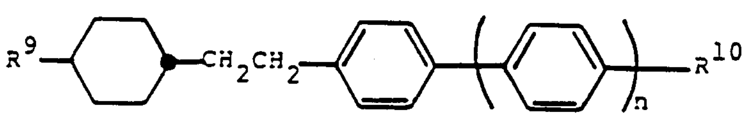

XXIV

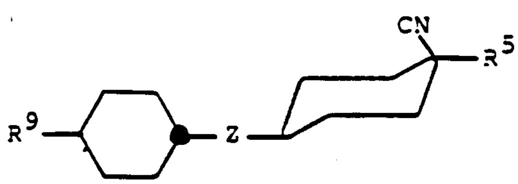

XXV

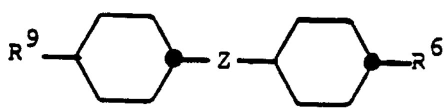

XXVI

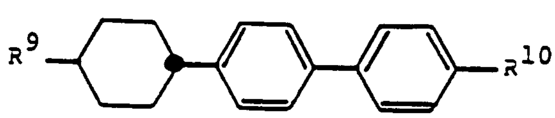

XXVII

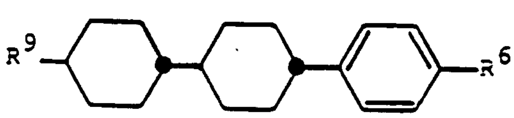

XXVIII

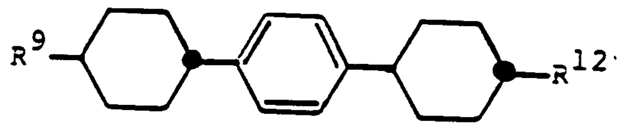

XXIX

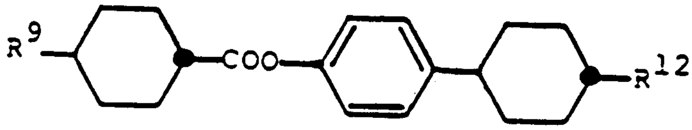

XXX

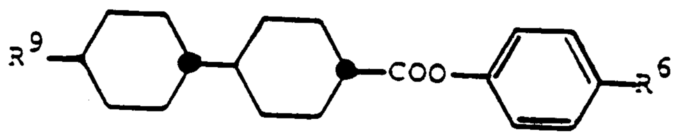

XXXI

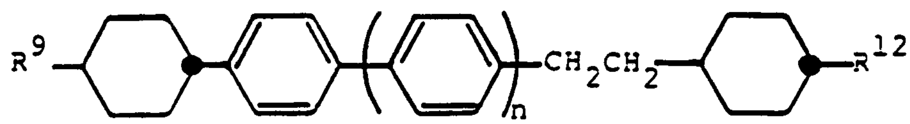

XXXII

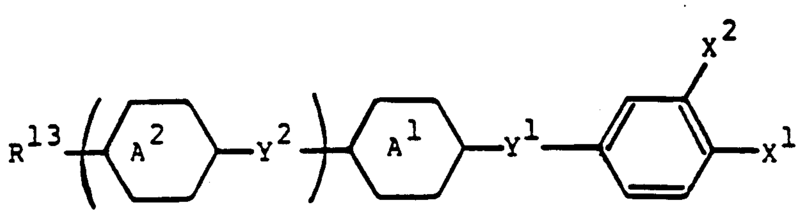

XXXIII

worin $R^5$ Alkyl, 3E-Alkenyl oder 4-Alkenyl bedeutet: $R^6$ Cyano, Alkyl, 1E-Alkenyl, 3E-Alkenyl, 4-Alkenyl, Alkoxy, 2E-Alkenyloxy oder 3-Alkenyloxy darstellt: $R^7$ und $R^8$ Alkyl oder Alkoxy bezeichnen: $R^9$ und $R^{12}$ unabhängig voneinander Alkyl, 1E-Alkenyl, 3E-Alkenyl oder 4-Alkenyl bedeuten; $A^3$ eine einfache Kovalenzbindung, 1,4-Phenylen oder trans-1,4-Cyclohexylen darstellt; $R^{10}$ Cyano, -NCS, Alkyl, 1E-Alkenyl, 3E-Alkenyl, 4-Alkenyl, Alkoxy, 2E-Alkenyloxy oder 3-Alkenyloxy bezeichnet; $R^{11}$ Alkoxy, 2E-Alkenyloxy oder 3-Alkenyloxy bedeutet: n für die Zahl 0 oder 1 steht; Z eine einfache Kovalenzbindung oder $-CH_2CH_2-$ darstellt: $X^1$ Fluor oder Chlor und $X^2$ Wasserstoff, Fluor oder Chlor bezeichnen: $R^{13}$ Alkyl, 3E-Alkenyl, 4-Alkenyl, Alkoxy, 2E-Alkenyloxy oder 3-Alkenyloxy bedeutet: eine der Gruppen $Y^1$ und $Y^2$ eine einfache Kovalenzbindung, -COO-, -OOC-, $-CH_2CH_2-$, $-CH_2O-$ oder $-OCH_2-$ und die andere der Gruppen $Y^1$ und $Y^2$ eine einfache Kovalenzbindung bedeutet; und die Ringe $A^1$ und $A^2$ unabhängig voneinander substituiertes oder unsubstituiertes trans-1,4-Cyclohexylen, in welchem gegebenenfalls 2 nicht benachbarte $CH_2$-Gruppen durch Sauerstoff ersetzt sind, oder substituiertes oder unsubstituiertes 1,4-Phenylen, in welchem gegebenenfalls 1 oder 2 $CH_2$-Gruppen durch Stickstoff ersetzt sind, darstellen, enthält.

**9.** Verfahren zur Herstellung der Verbindungen der in Anspruch 1 definierten Formel I, dadurch gekennzeichnet, dass man eine Verbindung der allgemeinen Formel

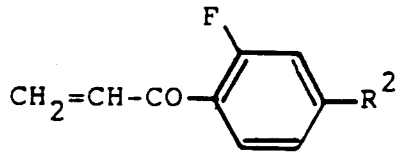

II

worin $R^2$ die in Anspruch 1 angegebene Bedeutung hat. mit einem Piperidin-Derivat der allgemeinen Formel

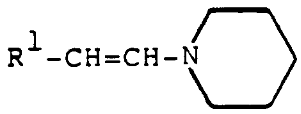

III

worin $R^1$ die in Anspruch 1 angegebene Bedeutung hat und anschliessend mit Hydroxylamin in Gegenwart von Säure umsetzt.

**10.** Verwendung der Verbindungen der in Anspruch 1 definierten Formel I für elektro-optische Zwecke.

**Claims**

**1.** Compounds of the general formula

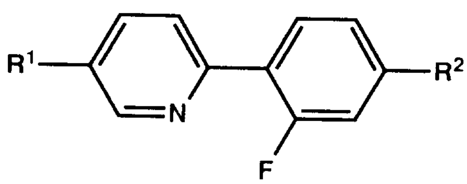

I

wherein $R^1$ signifies $(C_1\text{-}C_{18})$-alkyl or trans-4-$(C_1\text{-}C_{18}$-alkyl)cyclohexyl and $R^2$ signifies $(C_1\text{-}C_{18})$-alkyl, $(C_1\text{-}C_{18})$-alkoxy or trans-4-$(C_1\text{-}C_{18}$-alkyl)cyclohexyl.

**2.** Compounds of formula I according to claim 1, characterized in that $R^1$ signifies $(C_1\text{-}C_{12})$-alkyl or trans-4-$(C_1\text{-}C_{12}$-alkyl)cyclohexyl.

**3.** Compounds of formula I according to claim 1 or 2, characterized in that $R^2$ signifies $(C_1\text{-}C_{12})$-alkyl, $(C_1\text{-}C_{12})$-alkoxy or trans-4-$(C_1\text{-}C_{12}$-alkyl)cyclohexyl.

**4.** Compounds of formula I according to any one of claims 1, 2 or 3, characterized in that $R^1$ signifies $(C_1\text{-}C_7)$-alkyl or trans-4-$(C_1\text{-}C_7$-alkyl)cyclohexyl and $R^2$ signifies $(C_1\text{-}C_7)$-alkoxy.

**5.** A liquid crystalline mixture having at least 2 components, characterized in that at least one component is a compound of formula I defined in claim 1.

**6.** A liquid crystalline mixture according to claim 5, characterized in that it contains 1-60 wt.% of compounds of formula I defined in claim 1.

**7.** A nematic or cholesteric mixture according to claim 5 or 6, characterized in that it contains one or more compounds of formula I and one or more compounds having positive dielectric anisotropy.

**8.** A nematic or cholesteric mixture according to any one of claims 5 to 7, characterized in that it contains one or more compounds of formula I defined in claim 1 and or more compounds from the group of compounds of the general formulae

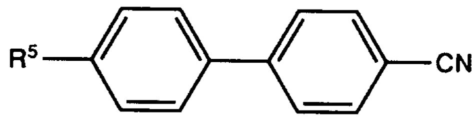

XVI

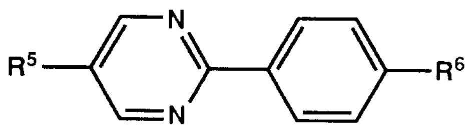

XVII

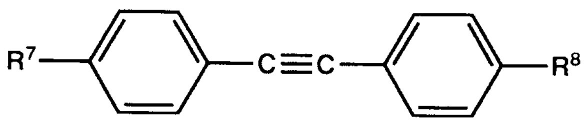

XVIII

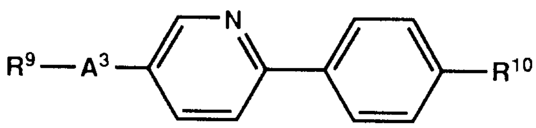

XIX

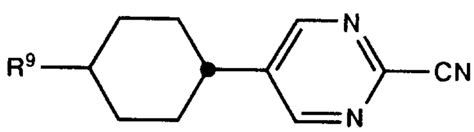

XX

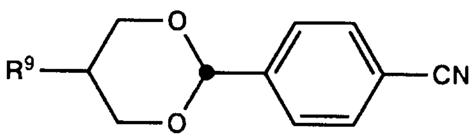

XXI

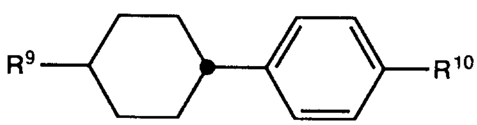

XXII

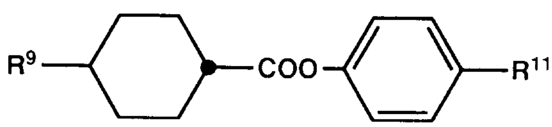

XXIII

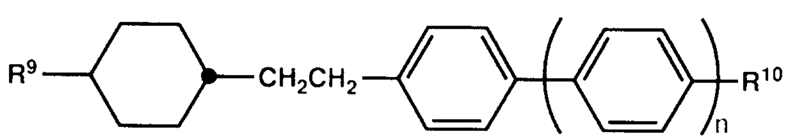

XXIV

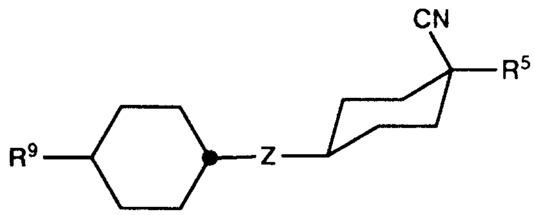

XXV

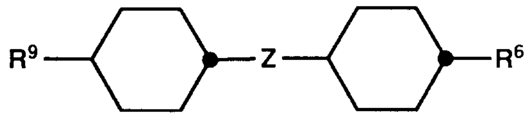

XXVI

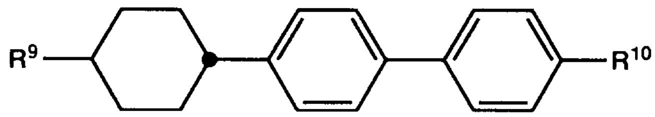

XXVII

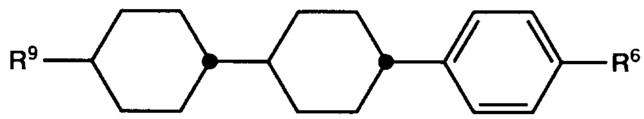

XXVIII

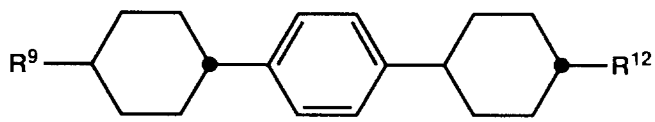

XXIX

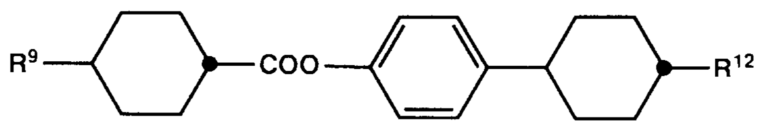

XXX

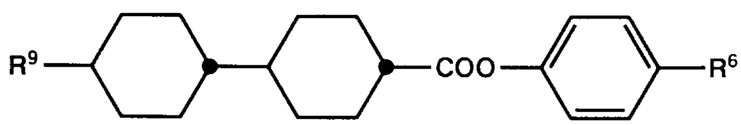

XXXI

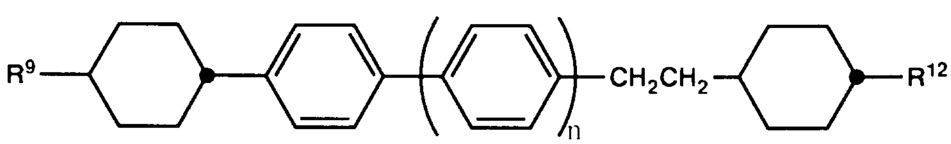

XXXII

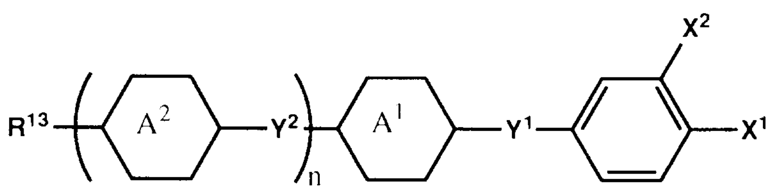

XXXIII

wherein $R^5$ signifies alkyl, 3E-alkenyl or 4-alkenyl; $R^6$ represents cyano, alkyl, 1E-alkenyl, 3E-alkenyl, 4-alkenyl, alkoxy, 2E-alkenyloxy or 3-alkenyloxy; $R^7$ and $R^8$ denote alkyl or alkoxy; $R^9$ and $R^{12}$ each independently signify alkyl, 1E-alkenyl, 3E-alkenyl or 4-alkenyl; $A^3$ represents a single covalent bond, 1,4-phenylene or trans-1,4-cyclohexylene; $R^{10}$ denotes cyano, -NCS, alkyl, 1E-alkenyl, 3E-alkenyl, 4-alkenyl, alkoxy, 2E-alkenyloxy or 3-alkenyloxy; $R^{11}$ signifies alkoxy, 2E-alkenyloxy or 3-alkenyloxy; n stands for the number 0 or 1; Z represents a single covalent bond or $-CH_2CH_2-$; $X^1$ denotes fluorine or chlorine and $X^2$ denotes hydrogen, fluorine or chlorine; $R^{13}$ signifies alkyl, 3E-

alkenyl, 4-alkenyl, alkoxy, 2E-alkenyloxy or 3-alkenyloxy; one of the groups $Y^1$ and $Y^2$ signifies a single covalent bond, -COO-, -OOC-, $-CH_2CH_2-$, $-CH_2O-$ or $-OCH_2-$ and the other of the groups $Y^1$ and $Y^2$ signifies a single covalent bond; and rings $A^1$ and $A^2$ each independently signify substituted or unsubstituted trans-1,4-cyclohexylene, in which optionally 2 non-adjacent $CH_2$ groups are replaced by oxygen, or substituted or unsubstituted 1,4-phenylene, in which optionally 1 or 2 $CH_2$groups is/are replaced by nitrogen.

**9.** A process for the manufacture of the compounds of formula I defined in claim 1, characterized by reacting a compound of the general formula

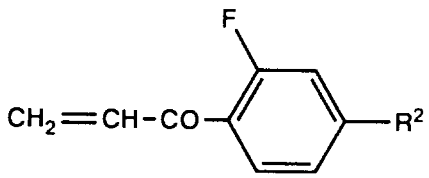

II

wherein $R^2$ has the significance given in claim 1, with a piperidine derivative of the general formula

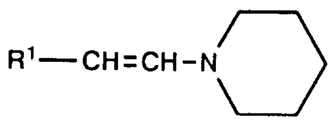

III

wherein $R^1$ has the significance given in claim 1, and subsequently with hydroxylamine in the presence of acid.

**10.** The use of the compounds of formula I defined in claim 1 for electro-optical purposes.

**Revendications**

**1.** Composés de formule générale

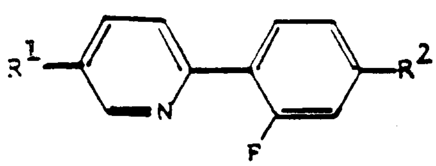

I

où $R^1$ représente un groupe alkyle($C_1$-$C_{18}$) ou trans-4-(alkyl en $C_1$-$C_{18}$)cyclohexyle et $R^2$ est un groupe alkyle($C_1$-$C_{18}$), alkoxy($C_1$-$C_{18}$) ou trans-4-(alkyl en $C_1$-$C_{18}$)cyclohexyle.

**2.** Composés de formule I selon la revendication 1, caractérisés en ce que $R^1$ représente un groupe alkyle($C_1$-$C_{12}$) ou trans-4-(alkyl en $C_1$-$C_{12}$)cyclohexyle.

**3.** Composés de formule I selon l'une des revendications 1 ou 2, caractérisés en ce que $R^2$ représente un groupe alkyle($C_1$-$C_{12}$), alcoxy($C_1$-$C_{12}$) ou trans-4-(alkyl en $C_1$-$C_{12}$)cyclohexyle.

**4.** Composés de formule I selon l'une quelconque des revendications 1, 2 ou 3, caractérisés en ce que $R^1$ représente un groupe alkyle($C_1$-$C_7$) ou trans-4-(alkyl en $C_1$-$C_7$)cyclohexyle et $R^2$ désigne un groupe alcoxy($C_1$-$C_7$).

**5.** Mélange en cristaux liquides ayant au moins 2 composants, caractérisé en ce qu'au moins un composant est un composé de formule I définie dans la revendication 1.

6. Mélange en cristaux liquides selon la revendication 5, caractérisé en ce qu'il contient 1-60% en poids de composés de formule I définie dans la revendication 1.

7. Mélange nématique ou cholestérique selon l'une des revendications 5 ou 6, caractérisé en ce qu'il contient un ou plusieurs composés de formule I et un ou plusieurs composés ayant une anisotropie diélectrique positive.

8. Mélange nématique ou cholestérique selon l'une quelconque des revendications 5 à 7, caractérisé en ce qu'il contient un ou plusieurs composés de formule I définie dans la revendication 1 et un ou plusieurs composés choisis dans le groupe des composés de formules générales

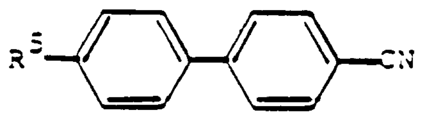

XVI

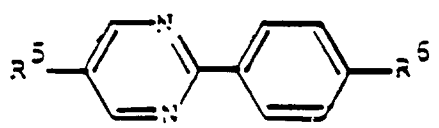

XVII

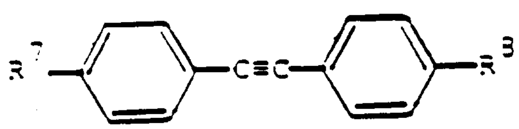

XVIII

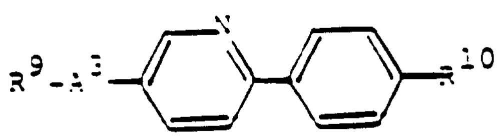

XIX

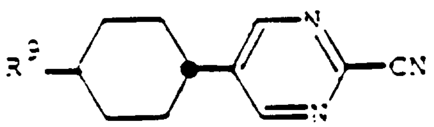

XX

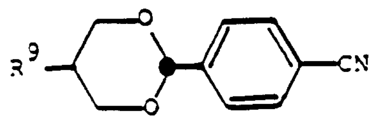

XXI

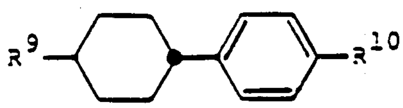

XXII

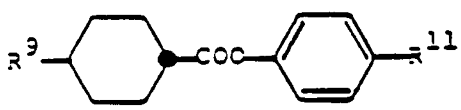

XXIII

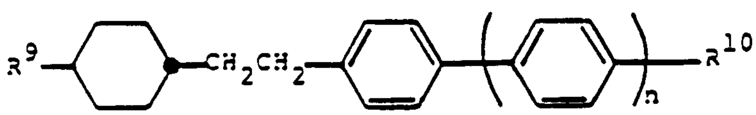

XXIV

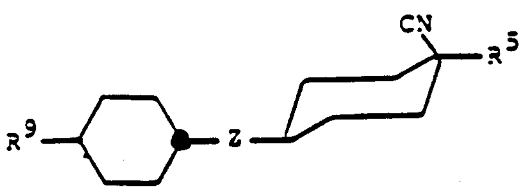

XXV

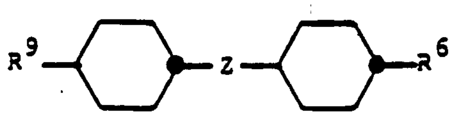

XXVI

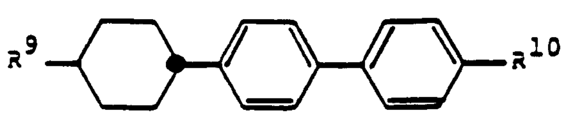

XXVII

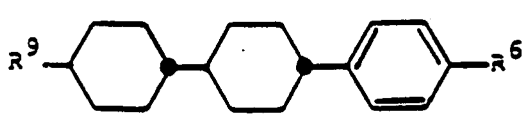

XXVIII

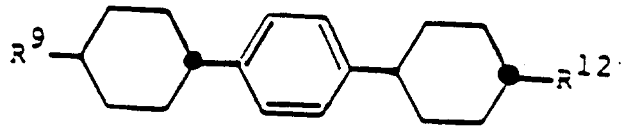

XXIX

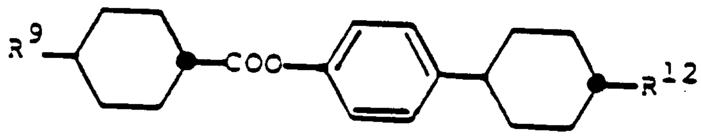

XXX

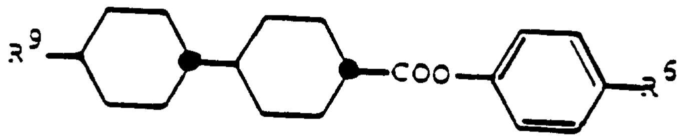

XXXI

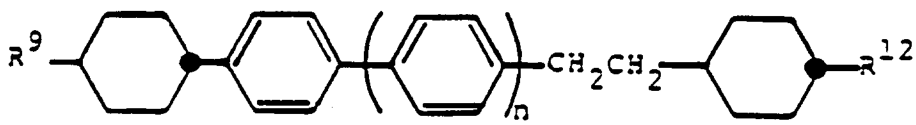

XXXII

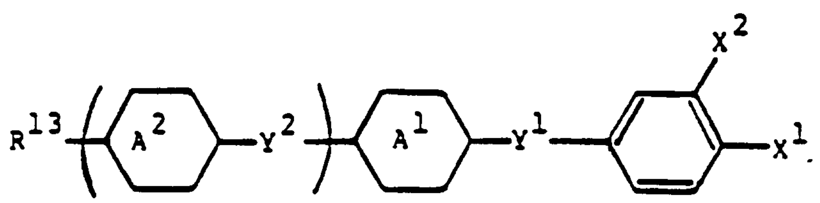

XXXIII

où $R^5$ représente un groupe alkyle, 3E-alcényle ou 4-alcényle; $R^6$ désigne un groupe cyano, alkyle, 1E-alcényle, 3E-alcényle, 4-alcényle, alcoxy, 2E-alcényloxy ou 3-alcényloxy; $R^7$ et $R^8$ désignent un groupe alkyle ou alcoxy; $R^9$ et $R^{12}$ représentent, indépendamment l'un de l'autre, un groupe alkyle, 1E-alcényle, 3E-alcényle ou 4-alcényle; $A^3$ désigne une liaison de covalence simple ou un groupe 1,4-phénylène ou trans-1,4-cyclohexylène; $R^{10}$désigne un groupe cyano, -NCS, alkyle, 1E-alcényle, 3E-alcényle, 4-alcényle, alcoxy, 2E-alcényloxy ou 3-alcényloxy; $R^{11}$ désigne un groupe alcoxy, 2E-alcényloxy ou 3-alcényloxy; n représente le nombre 0 ou 1; Z représente une liaison de covalence simple ou $-CH_2CH_2-$, $X^1$ désigne le fluor ou le chlore et $X^2$ désigne l'hydrogène, le fluor ou le chlore; $R^{13}$ représente un groupe alkyle, 3E-alcényle, 4-alcényle, alcoxy, 2E-alcényloxy ou 3-alcényloxy; l'un des groupes $Y^1$ et $Y^2$ représente une liaison de covalence simple, -COO-, -OOC-, $-CH_2CH_2-$, $-CH_2O-$ ou $-OCH_2-$ et l'autre des groupes $Y^1$ et $Y^2$ désigne une liaison de covalence simple; et les cycles $A^1$ et $A^2$ représentent, indépendamment l'un de l'autre, un groupe trans-1,4-cyclohexylène substitué ou non substitué, dans lequel éventuellement 2 groupes $CH_2$ non contigus sont remplacés par de l'oxygène, ou 1,4-phénylène substitué ou non substitué, dans lequel éventuellement 1 ou 2 groupes $CH_2$ sont remplacés par de l'azote.

**9.** Procédé pour la préparation de composés de formule I définie dans la revendication 1, caractérisé en ce qu'on fait réagir un composé de formule générale

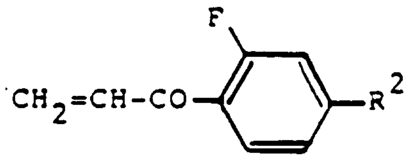

II

où $R^2$ a la signification indiquée dans la revendication 1, avec un dérivé de pipéridine de formule générale

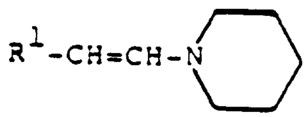

III

où $R^1$ a la signification indiquée dans la revendication 1 et ensuite avec de l'hydroxylamine en présence d'acide.

**10.** Utilisation des composés de formule I définie dans la revendication 1 à des fins électro-optiques.